# EUROPEAN PATENT SPECIFICATION

(11) **EP 3 745 991 B1**
(45) Date of publication and mention of the grant of the patent: **21.08.2024**
(21) Application number: 19748460.3
(22) Date of filing: 29.01.2019
(51) Int. Cl.: A61C 7/08, A61C 7/00, G16C 10/00

(54) **METHOD OF FORMING AN ELASTIC ORTHODONTIC APPLIANCE**
VERFAHREN ZUR HERSTELLUNG EINES ELASTISCHEN KIEFERORTHOPÄDISCHEN GERÄTES
MÉTHODE DE FABRICATION D'UN APPAREIL ORTHODONTIQUE ÉLASTIQUE

(30) Priority: 31.01.2018 US 201862624281 P; 07.03.2018 US 201815914932
(43) Date of publication of application: 09.12.2020
(73) Proprietor: BioTech Innovations, Inc., Norwalk, CA 90650 (US)
(72) Inventor: MOON Won, Los Angeles, CA 90049 (US); ABDELBAR Eheb, Plano, TX 75093 (US); KIM Paul, Diamond Bar, CA 91765 (US)
(74) Representative: Murgitroyd & Company
(86) International application number: PCT/US2019/015639
(87) International publication number: WO 2019/152396

(56) References cited:
- WO-A1-2017/006176
- US-A1- 2005 003 318
- US-A1- 2005 100 853
- US-A1- 2011 039 223
- US-A1- 2014 080 083
- US-A1- 2016 310 236
- US-A1- 2017 367 792

## Description

### FIELD OF THE INVENTION

The present invention generally relates to a method of forming an orthodontic aligning appliance.

### BACKGROUND OF THE INVENTION

Orthodontic tooth movement is accomplished by applying force to a tooth or set of teeth. This force causes a biological remodeling of the bone surrounding the teeth. This remodeling causes the tooth to shift and move inside the jaw bones. This orthodontic force can be applied by fixed and/or removable appliances. One type of appliances that is gaining popularity is orthodontic aligners or clear aligners. Clear aligners are a type of removable appliances used to move the teeth by having the patient wear the said aligner for 1-2 weeks and then switching to another sequential tray where they wear it for 1-2 weeks and so on and so forth.

Clear aligners are fabricated by thermoforming a heat sensitive clear plastic sheet onto a dental model of the teeth. In the aforementioned dental model, the teeth are shifted by a small increment in position and orientation. The concept is that the when the patient wears the plastic trays they will flex and distort and apply force to the teeth forcing them into a new position similar to the dental model used to fabricate the tray. The process is repeated for the number of trays needed to place the teeth in an ideal position. For example document US 2011/039223 A1 shows a method of forming an orthodontic aligner using a thermoset elastomer including reinforcement fibres.

There are a few inherent problems with plastic aligners. The current plastic tooth re-alignment technologies using clear aligners exert intermittent aligning forces, and thus, requires at least several trays, and often times more than a dozen trays, and lengthy treatment time (about 2 years) to re-align the teeth to a desired position, resulting in waste of tray materials and onerous doctor visit times so as to make the technology uneconomical and sometimes cost prohibitive. Moreover, clear Aligners have a limited range of flexing before they are permanently deformed and hence they can only move the teeth by a small increment at a time. If a large incremental movement is attempted, then usually two things will occur, which are either: 1) aligners will lose track of the teeth and will not move them into the proposed position or orientation, or 2) the aligner will distort beyond plastic deformation and will be permanently deformed and might not fit into the patient's mouth all together. Additionally, the nature of the material used in the aligner dictates the amount of force applied on the teeth and the rate at which the said force is dissipated. The plastic used in aligners applies a large amount of force initially and then it dissipates quickly. This is mainly due to the low flexibility of the material.

Further, the aforementioned force loading and dissipation is not biologically favorable in efficient tooth movement because it is considered a large intermittent force loading where teeth move faster and respond better to small continuous forces.

Therefore, there is a need for aligners that overcome the aforementioned shortcomings of the clear aligner.

Therefore, the embodiments described below address the above-identified issues and needs.

Because Aligners have a uniform thickness on the occlusal surface of the teeth. This can cause the posterior teeth to get imbedded inside the jaw bone (Intrusion movement). This is a general side effect of aligners that is usually very apparent with longer treatment times.

### SUMMARY OF THE INVENTION

The present invention defines a method of forming an orthodontic appliance, comprising both an elastic material and a reinforcing material,
wherein the orthodontic appliance comprises tooth sockets that correspond to each tooth on the dental arch,
the elastic material being in an amount that imparts to the appliance elastic properties such that the appliance exerts a continuous aligning force on a tooth or teeth along a dental arch;
wherein the elastic material accounts for all or substantially all the elastic properties of the appliance,
wherein the reinforcing material being polyester fibers or polyethylene fibers;
wherein the elastic material comprises at least 10 wt % of the appliance forming materials;
wherein the elastic material is other than polyester;
wherein the elastic material has an elasticity such that it is capable of being stretched at least 300% of its original length;
comprising:
   creating a dental model of a patient tooth and bite,
   constructing individual teeth from the dental model,
   moving a tooth or teeth of target of alignment according to a prescription by a treating doctor to create an aligned dental model of the patient,
   forming at least one appliance from the aligned dental model of the patient;
   forming the appliance by casting comprising:
      3D printing a cast-able appliance using a cast-able material,
      fabricating a mold from the cast-able appliance, and
      injecting into the mold the appliance forming material to form the appliance.

In some embodiments of the invention method, optionally in combination with any or all the various embodiments disclosed herein, moving a tooth or teeth of target comprises moving the tooth or teeth of target manually or using computer software.

In some alternative embodiments which are not part of the claimed invention, forming an appliance comprises forming the appliance by direct 3D printing (Additive manufacturing), CNC Machining.

In the method of the invention, the reinforcing material is to enhance appliance stability, anchorage, and force loading.

The description presents also an orthodontic appliance formed of a material (appliance forming material) comprising an elastic material in an amount that imparts to the aligner elastic properties such that the appliance exerts a continuous aligning force on a tooth or teeth, wherein the elastic material is a material other than polyester, and wherein the elastic material accounts for all or substantially all the elastic properties of the appliance.

In the appliance formed with the method of the present invention, optionally in combination with any or all the various embodiments disclosed herein, the elastic material comprises at least 10 wt% of the appliance forming material.

In some embodiments the appliance, optionally in combination with any or all the various embodiments disclosed herein, the elastic material comprises at least 20 wt% of the appliance forming material.

In some embodiments the appliance, optionally in combination with any or all the various embodiments disclosed herein, the elastic material comprises at least 30 wt% of the appliance forming material.

In some embodiments the appliance, optionally in combination with any or all the various embodiments disclosed herein, he elastic material comprises at least 40 wt% of the appliance forming material.

In some embodiments the appliance, optionally in combination with any or all the various embodiments disclosed herein, he elastic material comprises at least 50 wt% of the appliance forming material.

In some embodiments the appliance, optionally in combination with any or all the various embodiments disclosed herein, the elastic material comprises at least 60 wt% of the appliance forming material.

In some embodiments the appliance, optionally in combination with any or all the various embodiments disclosed herein, the elastic material comprises at least 70 wt% of the appliance forming material.

In some embodiments the appliance, optionally in combination with any or all the various embodiments disclosed herein, the elastic material comprises at least 80 wt% of the appliance forming material.

In some embodiments the appliance, optionally in combination with any or all the various embodiments disclosed herein, the elastic material comprises at least 90 wt% of the appliance forming material.

In some embodiments the appliance, optionally in combination with any or all the various embodiments disclosed herein, the elastic material comprises at least 95 wt% of the appliance forming material.

In some embodiments the appliance, optionally in combination with any or all the various embodiments disclosed herein, the elastic material comprises between 0-100 %. e.g. 0 < x < 100, where x is the elasticity.

In the appliance formed with the method of the present invention, optionally in combination with any or all the various embodiments disclosed herein, the elastic material has an elasticity that it is capable of being stretched at least 300% of its original length.

In some embodiments the appliance, optionally in combination with any or all the various embodiments disclosed herein, the elastic material is selected from the group consisting of silicone or orthodontic power chain material, and orthodontic rubber bands.

In the appliance formed with the method of the present invention, optionally in combination with any or all the various embodiments disclosed herein, the reinforcing material included in the appliance forming material is to enhance stability and anchorage of the appliance.

The appliance formed with the method of the present invention is used in a method of aligning a tooth or teeth of target of a patient, comprising: applying the appliance to the patient to move the tooth or teeth of target from an original position(s) to a prescribed position(s) according to a prescription by a treating doctor.

In some embodiments of the method, optionally in combination with any or all the various embodiments disclosed herein, the prescribed position is an intermediate position toward a final prescribed alignment of the tooth or teeth of target of alignment prescribed by a treating doctor.

In a further aspect of the present description, it is provided an orthodontic aligner kit, comprising at least one appliance according to the various embodiments disclosed herein.

In some embodiments of the aligner kit, optionally in combination with any or all the various embodiments disclosed herein, the aligner comprises a plurality of appliances, wherein each appliance is formed by a method according to the various embodiments of method of invention.

### DESCRIPTION OF THE DRAWINGS

Figure I is an image of an embodiment of an appliance, that can be formed with the method of the present invention, showing a view of the top side (non-tooth contacting side).
Figure 2 is an image of an embodiment of the appliance showing a view of the bottom side (tooth/teeth contacting side).
Figure 3 shows an image of an embodiment of the appliance (top) being applied to teeth (bottom).
Figures 4A-4D show one aspect of the appliance over traditional aligner appliances.

### DETAILED DESCRIPTION OF THE INVENTION

### Definitions

As used herein, the term "original position" of tooth or teeth refers to the position of the tooth or teeth of the target of treatment prior to the treatment using the appliance of invention; and the term "final position" refers to position of such tooth or teeth at the conclusion time point of an orthodontic treatment course prescribed by a treating doctor; and the term "intermediate position" refers to any position between the original position and the final position of the tooth or teeth of the target of treatment prescribed by a treating doctor.

As used herein, the term "appliance" or "aligner", which is used interchangeably herein, refers to an orthodontic device for moving a tooth or teeth according to a prescription by a treating doctor. Clear orthodontic appliances refer to those made from a plastic material, which is generally a polyester material. Such clear appliances are exemplified by Invisalign^{™} aligners. The Invisalign^{™} aligners, and methods of making and using the same, are described in general in U.S. Patent Nos. Pat. Nos. 6,450,807, and 5,975,893.

As used herein, the term "elastic appliance" refers to an orthodontic appliance capable of being stretched to a substantially higher degree than traditional polymer based clear aligners, such as the Invisalign^{™} aligners.

As used herein, the term "intermittent aligning force" refers to an aligning force generated by an aligner that substantially or entirely loses its strength due to characteristics of the material forming the aligner, e.g., due to fatigue, aging, degradation, erosion, or breaking down of the material. In contrast, "continuous aligning force" refers to an aligning force that is not intermittent, an example of "intermittent aligning force" being the aligning force generated by conventional plastic orthodontic aligners such as the Invisalign^{™} aligners.

### Orthodontic Appliances

The description presents an orthodontic appliance formed of a material (appliance forming material) comprising an elastic material in an amount that imparts to the aligner elastic properties such that the appliance exerts a continuous aligning force on a tooth or teeth, wherein the elastic material is a material other than polyester, and wherein the elastic material accounts for all or substantially all the elastic properties of the appliance.

In the appliance formed with the method of the present invention, optionally in combination with any or all the various embodiments disclosed herein, the elastic material comprises at least 10 wt% of the appliance forming material.

In some embodiments the appliance, optionally in combination with any or all the various embodiments disclosed herein, the elastic material comprises at least 20 wt% of the appliance forming material.

In some embodiments the appliance, optionally in combination with any or all the various embodiments disclosed herein, he elastic material comprises at least 30 wt% of the appliance forming material.

In some embodiments the appliance, optionally in combination with any or all the various embodiments disclosed herein, he elastic material comprises at least 40 wt% of the appliance forming material.

In some embodiments the appliance, optionally in combination with any or all the various embodiments disclosed herein, he elastic material comprises at least 50 wt% of the appliance forming material.

In some embodiments the appliance, optionally in combination with any or all the various embodiments disclosed herein, the elastic material comprises at least 60 wt% of the appliance forming material.

In some embodiments the appliance, optionally in combination with any or all the various embodiments disclosed herein, the elastic material comprises at least 70 wt% of the appliance forming material.

In some embodiments the appliance, optionally in combination with any or all the various embodiments disclosed herein, the elastic material comprises at least 80 wt% of the appliance forming material.

In some embodiments the appliance, optionally in combination with any or all the various embodiments disclosed herein, the elastic material comprises at least 90 wt% of the appliance forming material.

In some embodiments the appliance, optionally in combination with any or all the various embodiments disclosed herein, the elastic material comprises at least 95 wt% of the appliance forming material.

In some embodiments the appliance, optionally in combination with any or all the various embodiments disclosed herein, the elastic material comprises between 0-100 %. e.g. 0 < x < 100, where x is the elasticity.

In the appliance formed with the method of the present invention, optionally in combination with any or all the various embodiments disclosed herein, the elastic material has an elasticity that it is capable of being stretched at least 300% of its original length.

In some embodiments the appliance, optionally in combination with any or all the various embodiments disclosed herein, the elastic material is selected from the group consisting of silicone or orthodontic power chain material, and orthodontic rubber bands.

In the appliance formed with the method of the present invention, optionally in combination with any or all the various embodiments disclosed herein, the reinforcing material is to enhance stability and anchorage of the appliance.

### Appliance Forming Materials

Appliance forming materials usable for forming the elastic appliance can be any biocompatible elastic materials. As used herein, the term "elastic material" generally refers to materials which possess high elasticity (i.e.) which can be stretched several times its original length (at least 300%) and upon releasing of the stress (stretching force), it returns back to its original shape and dimensions.

Examples of elastic materials useful in the method of the present invention include elastomers, which are the class of polymer materials with high elastic nature. Elastomers show entropy-dependent elasticity. The polymer chains return back to the original state because it prefers to be in the lowest state of entropy. Elastomers are the class of polymer materials with high elastic nature and they include natural rubber, synthetic rubber, nitrile rubber, silicone rubber, urethane rubbers, chloroprene rubber, Ethylene Vinyl Acetate (EVA rubber), ethylene propylene diene monomer (M-class) rubber (EPDM), styrene-butadiene rubber (SBR), etc.

The appliance forming materials are substantially all elastic materials and include a reinforcing material, and additionally other materials, the elastic materials specifically exclude a polyester material.

As used herein, the term "reinforcing material" refers to any material capable of enhancing appliance stability, anchorage, and force loading. To enhance stability of an orthodontic appliance, such reinforcing material imparts attributes to the appliance that inhibits or prevents degradation or erosion of the appliance such that the appliance retains its elasticity and aligning force over the entire course of treatment or, alternatively, over a period of 2 weeks, 4 weeks, 6 weeks, 8 weeks, 12 weeks, 16 weeks, 20 weeks, 24 weeks, 26 weeks or 28 weeks, retaining at least 80% ( e.g., about 85%, 90%, 95%, 98%, or about 99%) of its elasticity and/or aligning force.

To enhance anchorage of an orthodontic appliance, such reinforcing material imparts, for example, attributes that allows the appliance to be anchored at a point or an area in the oral cavity. Such attributes can be, for example, adhesion, and thus, a reinforcing material that enhances adhesion of the appliance would be useful.

Such reinforcing materials used in the forming method of the invention include a fiber material, which fiber material is polyester fibers, or polyethylene fibers.

Optionally another reinforcing material such as for example metallic alloys used in orthodontics or dentistry in general (ST. steel, Ni Ti, TMA, Cu-Ni Ti) can be used.

The physical properties of the elastic material (modulus of elasticity, stiffness, etc.) can be readily modified according to the type of orthodontic movement needed with any specific case.

The appliance with elastic material will have the shape of individual teeth so that it fits around individual teeth. Using the analogy of glover-fingers, the appliance of invention fits around individual teeth like a glove fits individual fingers, and using the same analogy, previous art plastic aligners cover teeth like a mitten covers the fingers where all the fingers are wrapped around with no internal boundary. The appliance covers individual teeth and wrap around them to eliminate the tracking error problem of prior art.

In some embodiments, the internal dimensions of the elastic appliance can have the same size of the teeth to provide perfect fit or they can be designed to be slightly smaller, which is made possible due to the elasticity of the elastic appliance forming material. The combination of smaller size and elastic material will entail that the aligner will be slightly stretched and hence provide a better grip on individual teeth.

In some embodiments, the appliance can have very thin or no coverage in areas where upper and lower teeth interact or touch. This will eliminate the intrusion side effect mentioned earlier in the prior art.

In some further embodiments, the elastic appliance can have variable thickness either before fitting into the patient mouth or due to variable stretching that will happen after fitting into the patient mouth.

An advantage of the appliance is that it can be made to exert a relatively lighter force that will span for a longer time period before dissipating and another activation is needed. This provided a more biologically compatible and favorable force loading leading to more efficient and comfortable tooth movement.

### Aligner Kit

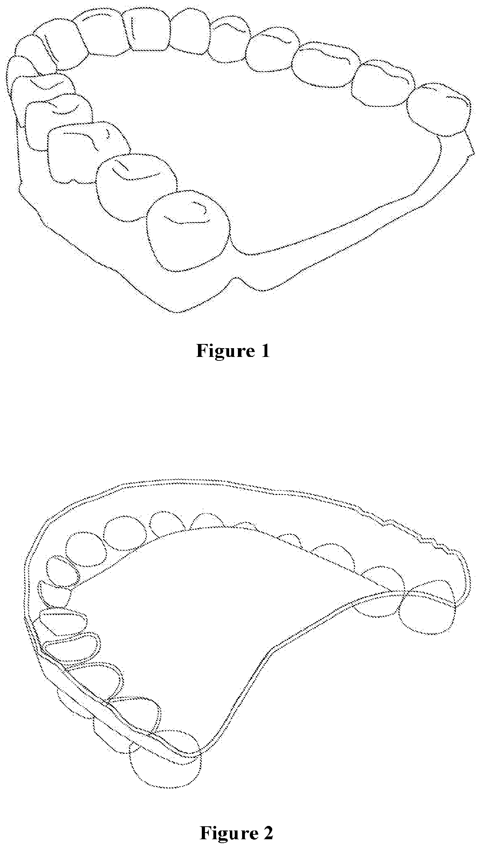

Figure 1 shows an image of an appliance showing a view of the top side (non-tooth contacting side) of the appliance. Figure 2 is an image of an embodiment of the appliance showing a view of the bottom side (tooth/teeth contacting side) of the appliance.

In a further aspect of the present description, it is provided an orthodontic aligner kit, comprising at least one appliance according to the various embodiments disclosed herein.

In some embodiments of the aligner kit, optionally in combination with any or all the various embodiments disclosed herein, the aligner comprises a plurality of appliances, wherein each appliance is formed by a method according to the various embodiments of the method of invention.

In some embodiments of the aligner kit, optionally in combination with any or all the various embodiments disclosed herein, the plurality of appliances have up to three appliances.

### Method of Fabrication

The present invention defines a method of forming an orthodontic appliance comprising both an elastic material and a reinforcing material,
wherein the orthodontic appliance comprises tooth sockets that correspond to each tooth on the dental arch,
the elastic material being in an amount that imparts to the appliance elastic properties such that the appliance exerts a continuous aligning force on a tooth or teeth along a dental arch;
wherein the elastic material accounts for all or substantially all the elastic properties of the appliance,
wherein the reinforcing material being polyester fibers or polyethylene fibers;
wherein the elastic material comprises at least 10 wt % of the appliance forming materials;
wherein the elastic material is other than polyester;
wherein the elastic material has an elasticity such that it is capable of being stretched at least 300% of its original length;
comprising:
   creating a dental model of a patient tooth and bite,
   constructing individual teeth from the dental model,
   moving a tooth or teeth of target of alignment according to a prescription by a treating doctor to create an aligned dental model of the patient,
   forming at least one appliance from the aligned dental model of the patient;
   forming the appliance by casting comprising:
      3D printing a cast-able appliance using a cast-able material,
      fabricating a mold from the cast-able appliance, and
      injecting into the mold the appliance forming material to form the appliance.

In some embodiments of the invention method, optionally in combination with any or all the various embodiments disclosed herein, moving a tooth or teeth of target comprises moving the tooth or teeth of target manually or using computer software.

In some embodiments not part of the claimed method, forming an appliance comprises forming the appliance by direct 3D printing.

In the invention the reinforcing material is to enhance appliance stability and anchorage.

3D-printing and casting are well documented methods of fabricating a 3D object.

In some embodiments of the invention method, optionally in combination with any or all the various embodiments disclosed herein, the elastic material comprises at least 20 wt% of the appliance forming material.

In some embodiments of the invention method, optionally in combination with any or all the various embodiments disclosed herein, he elastic material comprises at least 50 wt% of the appliance forming material.

In some embodiments of the invention method, optionally in combination with any or all the various embodiments disclosed herein, the elastic material comprises at least 80 wt% of the appliance forming material.

In some embodiments of the invention method, optionally in combination with any or all the various embodiments disclosed herein, the elastic material comprises at least 90 wt% of the appliance forming material.

In some embodiments of the invention method, optionally in combination with any or all the various embodiments disclosed herein, the elastic material comprises at least 95 wt% of the appliance forming material.

In some embodiments of the invention method, optionally in combination with any or all the various embodiments disclosed herein, the elastic material is selected from the group consisting of silicone or power chain material, and orthodontic rubber bands.

Methods of computer aided fabrication of orthodontic appliances are well documented in the art. Examples of such methods are described in U.S. Patent Nos. 6,450,807 and 5,975,893.

### Method of Use

The appliance formed with the method of the present invention is used in a method of aligning a tooth or teeth of target of a patient, comprising:
applying the appliance to the patient to move the tooth or teeth of target from an original position(s) to a prescribed position(s) according to a prescription by a treating doctor.

In some embodiments of the description, optionally in combination with any or all the various embodiments disclosed herein, the prescribed position is an intermediate position toward a final prescribed alignment of the tooth or teeth of target of alignment prescribed by a treating doctor.

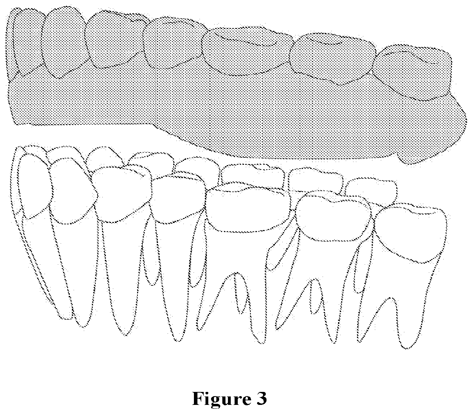

Figure 3 shows an image of an embodiment of the invention appliance (top) being applied to teeth (bottom).

The following examples illustrate embodiments which are not necessarily part of the present invention.

### Examples

### Example 1. 3D-printing fabrication of orthodontic appliance

An elastic orthodontic appliance was fabricated by 3D-printing using an appliance forming material using a method, which is not the claimed method, disclosed herein,
Figure 1 shows an image of the appliance viewed from the non-tooth contacting side, and Figure 2 shows an image of the appliance viewed from the tooth contacting side.

Figure 3 shows an image of an embodiment of the appliance (top) being applied to teeth (bottom).

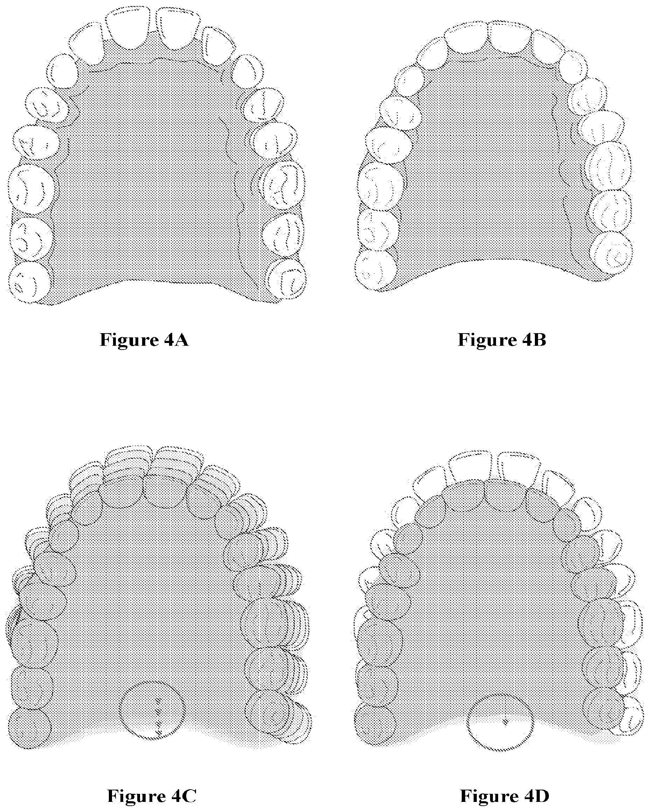

Figures 4A-4D show one aspect of the appliance over traditional aligner appliances in that the appliance significantly reduces the aligning steps in orthodontic treatment.

While various embodiments of the present invention have been shown and described herein, it will be obvious that such embodiments are provided by way of example only. Accordingly, it is intended that the invention be limited only by the wording of the appended claims.

## Claims

1. A method of forming an orthodontic appliance, comprising both an elastic material and a reinforcing material,
wherein the orthodontic appliance comprises tooth sockets that correspond to each tooth on the dental arch,
the elastic material being in an amount that imparts to the appliance elastic properties such that the appliance exerts a continuous aligning force on a tooth or teeth along a dental arch;
wherein the elastic material accounts for all or substantially all the elastic properties of the appliance;
wherein the reinforcing material being polyester fibers or polyethylene fibers; wherein the elastic material comprises at least 10 wt% of the appliance forming materials; wherein the elastic material is other than polyester; wherein the elastic material has an elasticity such that it is capable of being stretched at least 300% of its original length;
comprising:
creating a dental model of a patient tooth and bite, constructing individual teeth from the dental model,
moving a tooth or teeth of target of alignment according to a prescription by a treating doctor to create an aligned dental model of the patient,
forming at least one appliance from the aligned dental model of the patient; forming the appliance by casting comprising:
3D printing a cast-able appliance using a cast-able material,
fabricating a mold from the cast-able appliance, and
injecting into the mold the appliance forming material to form the appliance.

2. The method according to claim 1, wherein moving a tooth or teeth of target comprises moving the tooth or teeth of target manually or using computer software.

## Patentansprüche

1. Ein Verfahren zum Bilden eines kieferorthopädischen Geräts, das sowohl ein elastisches Material als auch ein Verstärkungsmaterial beinhaltet,
wobei das kieferorthopädische Gerät Zahnfächer beinhaltet, die jedem Zahn im Bereich des Zahnbogens entsprechen, das elastische Material in einer Menge vorliegt, die dem Gerät elastische Eigenschaften gibt, sodass das Gerät eine kontinuierliche Richtkraft auf einen Zahn oder Zähne entlang eines Zahnbogens aufbringt;
wobei das elastische Material alle oder im Wesentlichen alle der elastischen Eigenschaften des Geräts ausmacht;
wobei das Verstärkungsmaterial aus Polyesterfasern oder Polyethylenfasern besteht;
wobei das elastische Material zu mindestens 10 Gew.-% die Gerätbildungsmaterialien beinhaltet;
wobei das elastische Material aus etwas anderem als Polyester besteht;
wobei das elastische Material eine Elastizität aufweist, sodass es um mindestens 300 % seiner ursprünglichen Länge gedehnt werden kann;
beinhaltend:
Herstellen eines Zahnmodells eines Patientenzahns und -bisses,
Konstruieren individueller Zähne aus dem Zahnmodell,
Bewegen eines Zahns oder von Zähnen, die Ziel der Ausrichtung sind, gemäß einer Verschreibung durch einen behandelnden Arzt, um ein ausgerichtetes Zahnmodell des Patienten herzustellen,
Bilden mindestens eines Geräts aus dem ausgerichteten Zahnmodell des Patienten;
Bilden des Geräts durch Gießen, beinhaltend:
3D-Drucken eines gießfähigen Geräts unter Verwendung eines gießfähigen Materials,
Fertigen einer Form aus dem gießfähigen Gerät, und
Einspritzen des gerätbildenden Materials in die Form, um das Gerät zu bilden.

2. Verfahren gemäß Anspruch 1, wobei das Bewegen eines Zielzahns oder von -zähnen das Bewegen des Zielzahns oder der -zähne manuell oder unter Verwendung einer Computersoftware beinhaltet.

## Revendications

1. Un procédé de formage d'un appareil orthodontique, comprenant à la fois un matériau élastique et un matériau de renfort,
dans lequel l'appareil orthodontique comprend des alvéoles pour dents qui correspondent à chaque dent sur l'arcade dentaire, le matériau élastique étant présent en une quantité qui confère à l'appareil des propriétés élastiques telles que l'appareil exerce une force d'alignement continue sur une ou plusieurs dents le long d'une arcade dentaire ;
dans lequel le matériau élastique assure toutes ou substantiellement toutes les propriétés élastiques de l'appareil ;
dans lequel le matériau de renfort consiste en des fibres de polyester ou des fibres de polyéthylène ;
dans lequel le matériau élastique représente au moins 10 % en poids des matériaux de formage d'appareil ;
dans lequel le matériau élastique est un matériau autre que le polyester ;
dans lequel le matériau élastique présente une élasticité telle qu'il est capable d'être étiré d'au moins 300 % de sa longueur d'origine ;
comprenant :
la création d'un modèle dentaire de la/des dent(s) et de la morsure d'un patient,
la construction de dents individuelles à partir du modèle dentaire,
le déplacement d'une ou de plusieurs dents ciblée(s) pour un alignement selon une prescription par un médecin prescrivant le traitement afin de créer un modèle dentaire aligné du patient,
le formage d'au moins un appareil à partir du modèle dentaire aligné du patient ;
le formage de l'appareil par coulage comprenant :
l'impression 3D d'un appareil coulable en utilisant un matériau coulable,
la fabrication d'un moule à partir de l'appareil coulable, et
l'injection dans le moule du matériau de formage d'appareil afin de former l'appareil.

2. Le procédé selon la revendication 1, dans lequel le déplacement d'une ou de plusieurs dents ciblée(s) comprend le déplacement de la ou des dents ciblée(s) manuellement ou à l'aide d'un logiciel informatique.
